# EUROPEAN PATENT APPLICATION

(11) **EP 1 897 898 A1**
(43) Date of publication of application: **12.03.2008**
(21) Application number: 06746083.2
(22) Date of filing: 08.05.2006
(51) Int. Cl.: C08G 67/00, A61K 47/48, C08L 101/16

(54) **DEGRADABLE POLYMER AND PROCESS FOR PRODUCTION THEREOF**

(30) Priority: 09.05.2005 JP 2005136699
(71) Applicant: Japan Science and Technology Agency, Kawaguchi-shi, Saitama 332-0012 (JP); Osaka Industrial Promotion Organization, Osaka-shi, Osaka 540-0029 (JP)
(72) Inventor: MATSUMOTO, Akikazu, Kawachinagano-shi, 586-0077 Osaka (JP)
(74) Representative: Bittner, Thomas L.
(86) International application number: PCT/JP2006/309252
(87) International publication number: WO 2006/121007

(57) **Abstract**

Disclosed is a degradable polymer having a peroxide bond therein. The degradable polymer can be utilized in the fields of medicals or medical materials, can be applied to a DDS or gene delivery system, and can be used as a novel polymeric material or environmentally-friendly material. A polyperoxide (114), which is an alternating copolymer, has in a side chain thereof, a functional group (112) (which is a substituent comprising a therapeutic molecule), a water-soluble substituent, or a biodegradable substituent.

## Description

### TECHNICAL FIELD

The present invention relates to a degradable polymer and a method of producing the same. More specifically, for example, the present invention relates to a degradable polymer, which can be used as environmentally friendly or biocompatible novel polymer material, and a method of producing the same.

### BACKGROUND ART

While degradable polymers made of vinyl monomers or diene monomers have been intensively studied, there are a few examples of highly degradable vinyl polymers or diene polymers due to carbon-carbon bond connecting the their main chains. It was first suggested in 1920s to produce a polymer or oligomer having peroxide bonding via copolymerization with oxygen. In 1940s to 1950s, a quite number of researches were carried out on the production of such a polymer or oligomer. Since then, a number of studies have been reported by academic papers and the like. These arts synthesize peroxide polymers (polyperoxide) by reacting the vinyl monomer under high pressure and are inevitable from breaking down the synthesized polymer during the synthesis reaction. This results in unsteady degradation property of the polyperoxide, thereby to discourage using the polyperoxide and designing the polyperoxide as a polymer material. Especially, an easy method of producing an alternating copolymer of a diene monomer (such as a sorbic acid derivative, hexadiene, or the like) and oxygen has not been reported so far.

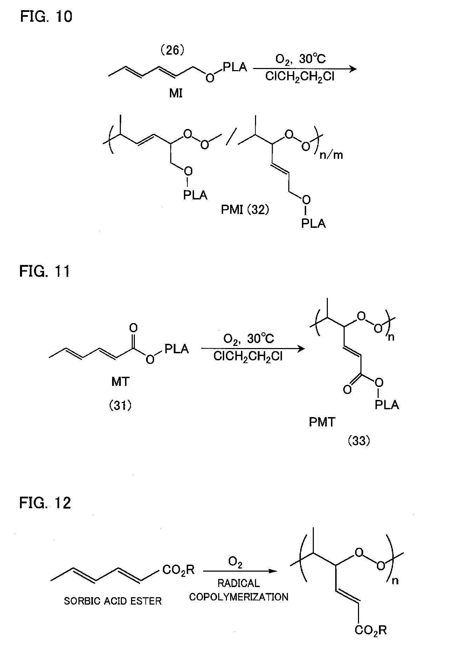

The inventor of the present invention found that it is possible to synthesize an alternating copolymer in a crystalline lattice by radical alternating polymerization of a diene monomer (sorbic acid ester) and oxygen by heat application or heat and light application as illustrated in Fig. 12 (see Non-Patent Citation 1 and Patent Citation 1, for example).

Meanwhile, synthesis of new environmentally-friendly polymer materials have been energetically studied in response to the environmental issues, which are much concerned recently. When biodegradable polymers are left underground, they are broken down to compounds of lower molecular weights by microorganisms and the like living therein. The compounds of lower molecular weights, which are then taken up into the metabolic cycles of microorganisms, are converted into carbon dioxide finally. A biodegradable polymer synthesized from a plant-origin or nature-origin raw material(s) can be a recyclable material that is taken into the carbon cycle in the nature. Biodegradable polymers play more important roles in the medical field. In the medical field, there are many applications in which materials to use (such as drug releasing devices controlling their releases by degradation, suture threads, bone fixation materials, in vivo hemostatic/adhering agents, adhesion inhibiting agents, tissue engineering materials, and the like) are desirably made of an *in vivo* degradable polymer, which, after completion of the duty thereof, is enzymatically or non-enzymatically hydrolyzed *in vivo* to metabolizalble or absorbable compounds.

For example, polylactic acid (PLA) is produced from lactic acid or lactide which is a cyclic lactic acid dimer obtainable from a starch of corn or the like. PLA is broken down to lactic acid, which is metabolically absorbable *in vivo* along the cycle o the nature. Thus, PLA is one of materials expected as recyclable materials. It is known that PLA is relatively excellent in biocompatibility and mechanical strength. Moreover, PLA can be co-polymerized with a cyclic monomer usable as a monomer in the polymerization but other than lactide. PLA, therefore, allows to easily improve or adjust a property of the material. Moreover, application of PLA to biocompatible material having a function-having functional group has been considered.

The material using PLA is relatively high in cost currently. Thus, PLA is utilized more favorably in the medical and medical engineering fields rather than in industrial application, which requires mass production. Specific examples of the use of PLA are biocompatible material, regenerative medical engineering material, microsphere for DDS (drug delivery system), and the like. Degradation rate is one of properties important in the application as the biodegradable polymer material. It is known that poly-L-lactic acid (PLLA) and poly-D-lactic acid (PDLA) having high crystallinities are hydrolyzed at relatively slow rates because the hydrolysis rate of PLA is influenced by crystallinity of the polymer. Its hydrophobic property and poor flexibility of PLA results in low compatibility thereof toward living soft tissues. Thus, the usage of PLA is still restricted. A number of molecular-level researches have been undertaken until today in order to solve the problems associated with the hydrophobic property and poor flexibility of PLA, yet keeping or improving the excellent properties of PLA, in order to broaden the applications of PLA as materials.

In general, the most biodegradable polymers have in its main chain a biodegradable (hydrolyzable) component such as ester, amide, carbonate, urea, glycoside bonding, or the like, and are broken down slowly over a few week or longer by an effect of an microorganism or the other effect. On the other hand, it is known that vinyl polymers except vinyl alcohols are poor in degradability. However, polyperoxides having plural peroxy bonds in its main chain is degradable in a short time at heat or light application as described above. Thus, the application of polyperoxides is expected to be different from that of the biodegradable polymers, which have been used as replacement of polymer materials and whose degradability aims to being environmentally friendly. For polyperoxides, the degradability thereof per se is its function.

No polyperoxide has been known, which is an alternating copolymer having a drug, hydrophilic group, biodegradable group, or the like in its side chain. Moreover, no polyperoxide has been known, which has a combination of such function-having groups of different properties.

### [Non-Patent Citation 1]

Akikazu MATSUMOTO, and one other "Radical Alternating Copolymerization of Sorbic Esters and Oxygen in the Solid State Under Photoirradiation", Digest of the 44th Annual Meeting of the society of polymer science, Japan, July 10, 1998, p13.

### [Patent Citation 1]

Pamphlet of International Publication No.2004/087791 (published on October 14, 2004)

### DISCLOSURE OF INVENTION

In order to attain the object, a degradable polymer according to the present invention is a degradable polymer having in its main chain a peroxide bond by radical alternating copolymerization of a diene monomer and oxygen, the degradable polymer including: a substituent in its side chain, the substituent having at least one function selected from the group consisting of a pharmacologic activity function, an optical function, an electronic function, an electric function, a magnetic function, a chiral recognizing function, a catalyst function, a liquid crystal function, an actuator function, and a sensor function.

It is preferable that the degradable polymer according to the present invention have a structure represented by General Formula (I): 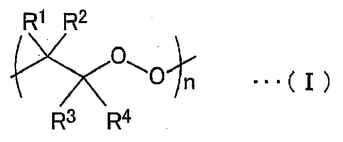 where R¹, R², and R³ are independently an alkyl group or an aromatic group, and R⁴ is a substituent having at least one function selected from the group consisting of the pharmacologic activity function, the optical function, the electronic function, the electric function, the magnetic function, the chiral recognizing function, the catalyst function, the liquid crystal function, the actuator function, and the sensor function, and n is any integer.

It is preferable that the degradable polymer according to the present invention have a structure represented by General Formula (II): 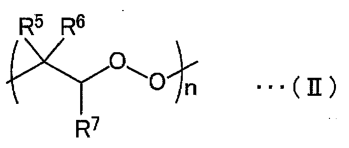 where R⁵ and R⁶ are independently an alkyl group or an aromatic group, and R⁷ is a substituent having at least one function selected from the group consisting of the pharmacologic activity function, the optical function, the electronic function, the electric function, the magnetic function, the chiral recognizing function, the catalyst function, the liquid crystal function, the actuator function, and the sensor function, and n is any integer.

It is preferable that the degradable polymer according to the present invention have a structure represented by General Formula (III): 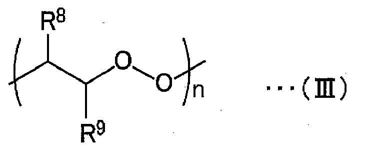 where R⁸ is an alkyl group or an aromatic group, and R⁹ is a substituent having at least one function selected from the group consisting of the pharmacologic activity function, the optical function, the electronic function, the electric function, the magnetic function, the chiral recognizing function, the catalyst function, the liquid crystal function, the actuator function, and the sensor function, and n is any integer.

Moreover, the degradable polymer is preferably arranged such that the substituent is a drug molecule.

Moreover, the degradable polymer is preferably arranged such that the substituent is water soluble.

Moreover, the degradable polymer is preferably arranged such that the substituent is biodegradable.

Moreover, the degradable polymer is preferably arranged such that the diene monomer includes a compound having at least two diene groups.

Moreover, the degradable polymer is preferably arranged such that it has a gel structure

A method according to the present invention is a method of producing a degradable polymer having in its main chain a peroxide bond by radical alternating copolymerization of a diene monomer and oxygen, the degradable polymer including a substituent in its side chain, the substituent having at least one function selected from the group consisting of a pharmacologic activity function, an optical function, an electronic function, an electric function, a magnetic function, a chiral recognizing function, a catalyst function, a liquid crystal function, an actuator function, and a sensor function, the method including: performing the radical alternating copolymerization after bonding the diene monomer with the substituent, so as to have the structure in which the degradable polymer includes the substituent in its side chain.

The method according to the present invention is preferably arranged such that the substituent is at least one selected from the group consisting of a substituent made of a drug molecule, a water soluble substituent, and a biodegradable substituent.

A method according to the present invention is a method of producing a degradable polymer having in its main chain a peroxide bond by radical alternating copolymerization of a diene monomer and oxygen, the degradable polymer including a substituent in its side chain, the substituent having at least one function selected from the group consisting of a pharmacologic activity function, an optical function, an electronic function, an electric function, a magnetic function, a chiral recognizing function, a catalyst function, a liquid crystal function, an actuator function, and a sensor function, the method including: bonding the substituent with the degradable polymer in its side chain after performing the radical alternating copolymerization of the diene monomer with the substituent.

The method according to the present invention is preferably arranged such that the substituent is at least one selected from the group consisting of a substituent made of a drug molecule, a water soluble substituent, and a biodegradable substituent.

A method according to the present invention is a method of producing a degradable polymer having in its main chain a peroxide bond by radical alternating copolymerization of a diene monomer and oxygen, the degradable polymer including a substituent in its side chain, the substituent having at least one function selected from the group consisting of a pharmacologic activity function, an optical function, an electronic function, an electric function, a magnetic function, a chiral recognizing function, a catalyst function, a liquid crystal function, an actuator function, and a sensor function, wherein: the diene monomer includes a compound having at least two diene groups.

These arrangements realize that the alternating copolymer has in its side chain the substituent, which is a drug, a hydrophilic group, a biodegradable group, or the other. This makes the alternating copolymer usable in the medical field and medical material field, applicable to DDS and gene delivery system, and adaptable to proving a novel polymer material and environmentally friendly material.

### BRIEF DESCRIPTION OF DRAWINGS

Fig. 1 is a view illustrating a synthetic reaction of a polyperoxide to which a function is provided.
Fig. 2 is a view illustrating how to introduce a reactive group into the polyperoxide thereby to give a function thereto.
Fig. 3 is a view illustrating examples of substituents bondable to the polyperoxide.
Fig. 4 is a view illustrating a synthetic reaction of a polyperoxide having a 5-FU in its side chain.
Fig. 5 is a view illustrating a synthetic reaction of a polyperoxide having a sugar in its side chain.
Fig. 6 is a view illustrating a synthetic reaction from a sugar and a diene monomer.
Fig. 7 is a view illustrating a synthetic reaction of a water soluble polyperoxide.
Fig. 8 is a graph illustrating a phase diagram of a copolymer.
Fig. 9 is a view illustrating a synthetic reaction from polylactic acid macromonomer.
Fig. 10 is a view illustrating a synthetic reaction of a polyperoxide prepared by copolymerization of polylactic acid macromonomer and oxygen.
Fig. 11 is a view illustrating a synthetic reaction of a polyperoxide prepared by copolymerization of polylactic acid macromonomer and oxygen.
Fig. 12 is a view illustrating a synthetic reaction of polyperoxide.
Fig. 13 is a graph illustrating how molecular weight of a polyperoxide was changed.
Fig. 14 is a view illustrating how outer appearance of a polyperoxide having a gel structure was changed before and after degradation thereof.
Fig. 15 is a view illustrating a synthetic reaction of a telechelic polymer.

### [Explanation of Referential Numerals]

111: Monomer
112: Functional Group
113: Polyperoxide
114: Polyperoxide to which a function is given

### BEST MODE FOR CARRYING OUT THE INVENTION

A degradable polymer according to one embodiment is a degradable polymer having a peroxide bond in its main chain, and being prepared by radical alternating copolymerization from a diene monomer and oxygen. The degradable polymer has a substituent in its side chain, the substituent having at least one function selected from the group consisting of a pharmacologic activity function, an optical function, an electronic function, an electric function, a magnetic function, a chiral recognizing function, a catalyst function, a liquid crystal function, an actuator function, and a sensor function. (Hereinafter, this substituent is referred to as "function-having substituent.")

The substituent having the pharmacological activity function is a substituent that has a pharmacological effect to react with a particular material or compound in vivo. For example, the substituent having the pharmacological activity function may be a substituent including a chemical structure of a component of a drug (such as medical drug (e.g. anti-cancer drug), a gene therapy drug, and the like). Moreover, the drug is not limited to applications as a medical drug and a poison and may be an oral drug, injectable solution, poultice, or a drug that is directly applied to a part of a body. Further, the substituent having the pharmacological activity function is not limited to substituents having a chemical structure of a component of a drug that has been used. The substituent having the pharmacological activity function encompasses substituents having any chemical structure that is pharmacologically active or interactive *in vivo.*

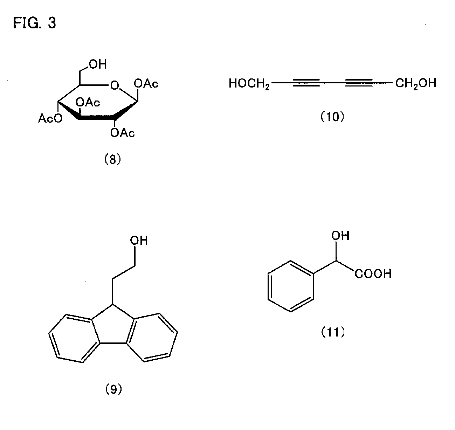

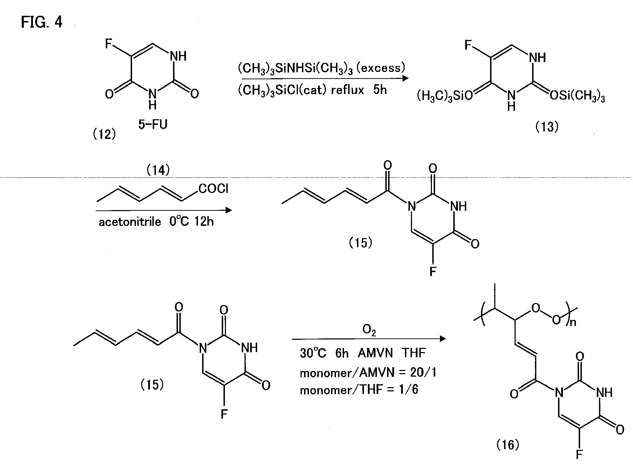

Specific examples of the substituent having the pharmacological activity function encompass substituents having structures of a compound (8) illustrated in Fig. 3 or a compound (12) illustrated in Fig. 4.

The substituent having the optical function is a substituent reactive with light of a particular wavelength. For example, the substituent having the optical function may be: a substituent that emits fluorescence by absorbing particular light; an optically reactive substituent that undergoes a reaction such as photochromism (to change color in response to light), thermochromism (to change color in response to heat), or photoisomerization, or the like reaction; a substituent having an electroluminescent property that emits light at application of electricity; a substituent having a non-linear optical property; a substituent functioning an optical memory; a substituent having a photolithographic function; and the like. One specific example of the substituent having the optical function is a substituent having the structure of compound (9) illustrated in Fig. 3 described later.

The substituent having the electronic function is a substituent that interacts with an electron or changes its electronic state, thereby to show a characteristic change. Examples of the substituent having the electronic function encompass a substituent having electroconductivity, electrochromism, piezo/pyroelectricity, photoelectric conversion, or the other electronic function. One specific example of the substituent having the electronic function is the substituent having the structure of the compound (9) illustrated in Fig. 3.

The substituent having the electric function is a substituent that works electrically or electrochemically. Examples of the substituent having the electric function encompass substituents having a function applicable to a chemical cell, secondary cell, capacitor or the like, or having ferroelectricity or the like. One specific example of the substituent having the electric function is a substituent having a structure of a compound (10) illustrated in Fig. 3.

The substituent having the magnetic function is a substituent that is interactive with magnet or is magnetically characteristic. Examples of the substituent having the magnetic function encompass a substituent, which is ferromagnetic or exhibits a high-spin state.

The substituent having the chiral recognizing function is substituent that reacts with a chiral compound thereby performing chiral recognition or thereby performing not only the chiral recognition but also separation of the chiral compound. Examples of the substituent having the chiral recognizing function encompass a substituent having a component or chemical structure of an optically separating reagent, optically separating column, or the like. One example of the substituent having the chiral recognizing function is a substituent having the structure of a compound (11) illustrated in Fig. 3.

The substituent having the catalyst function is a substituent that promotes a chemical reaction by a small quantity thereof, or that selectively catalyzes particular one of reactions. Examples of the substituent having the catalyst function encompass a substituent having a chemical structure of a catalyst for a chemical reaction encompassing oxidation, reduction, addition reaction, substitution reaction, polymerization, and the other reaction, and a substituent that exhibits a catalystic activity. The catalyst is not particularly limited and may be a catalyst categorized in homogeneous catalyst, heterogeneous catalyst, solid catalyst, or may be part of such a catalyst.

The substituent having the liquid crystal function is a substituent that exhibits thermotropic liquid crystallinity, lyotropic liquid crystallinity, cholesteric liquid crystallinity, or the other liquid crystallinity. Examples of the substituent having the liquid crystal function encompass a substituent having a liquid crystalline structure or component called mesogen, a substituent having a chemical structure of a liquid crystalline polymer or the like, and the like substituent.

The substituent having the actuator function is a substituent exhibiting a motor function by converting a physical energy (electricity, light, magnet, or the like) or a chemical energy into a mechanical energy.

The substituent having the sensor function is a substituent that qualitatively or quantitatively senses a particular substance such as an atom, a molecule, an ion, or the like, or a substituent that senses a physical change. The substituent having the sensor function has a function of an element that converts a physical value regarding a state or qualitative value of analyte, into another physical value that is easy to transfer, record, or process digitally. Examples of the substituent having the sensor function encompass a substituent that functions as a gas sensor, ion sensor, thermo sensor, pressure sensor, and the like. The function of the element encompasses a detector function, a transducer function, a converter function, and the like.

The function-having substituent is in the side chain of the degradable polymer directly or indirectly via a spacer by bonding such as covalent bonding, coordinate bonding, metal bonding, ionic bonding, hydrogen bonding, or the other bonding, or by an intermolecular interaction or spatial entrapment.

It is preferable that the degradable polymer according to the present embodiment have a structure represented by General Formula (I): 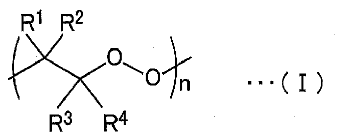 where R¹, R², and R³ are independently an alkyl group or an aromatic group, and R⁴ is a substituent having at least one function selected from the group consisting of the pharmacologic activity function, the optical function, the electronic function, the electric function, the magnetic function, the chiral recognizing function, the catalyst function, the liquid crystal function, the actuator function, and the sensor function, and n is any integer.

Meanwhile, it is also preferable that the degradable polymer according to the present embodiment have a structure represented by General Formula (II): 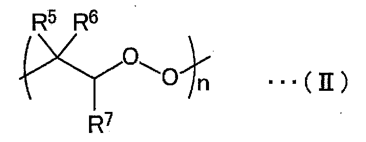 where R⁵ and R⁶ are independently an alkyl group or an aromatic group, and R⁷ is a substituent having at least one function selected from the group consisting of the pharmacologic activity function, the optical function, the electronic function, the electric function, the magnetic function, the chiral recognizing function, the catalyst function, the liquid crystal function, the actuator function, and the sensor function, and n is any integer.

Moreover, it is also preferable that the degradable polymer according to the present embodiment have a structure represented by General Formula (III): 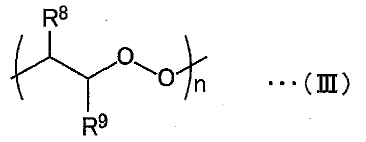 where R⁸ is an alkyl group or an aromatic group, and R⁹ is a substituent having at least one function selected from the group consisting of the pharmacologic activity function, the optical function, the electronic function, the electric function, the magnetic function, the chiral recognizing function, the catalyst function, the liquid crystal function, the actuator function, and the sensor function, and n is any integer.

The degradable polymer according to the present embodiment is adjustable in degradation temperature or degradation rate by changing the substituents other than the function-having substituent in number, kind, or the other. Thus, the degradation temperature or degradation rate can be controlled without prohibiting selecting an appropriate polyperoxide for usage and adopting any kind of the function-having group. For example, the use of the degradable polymer having the structure represented in General Formula (III) hardly causes degradation around 50°C. On the other hand, the use of the degradable polymer having the structure represented in General Formula (I) gives such an adequate degradation rate around 50°C that about a half or more is broken down in a few hours. Meanwhile, the use of the degradable polymer having the structure represented in General Formula (II) gives a degradation property intermediate between that of the degradable polymer having the structure represented in General Formula (I) and that of the degradable polymer having the structure represented in General Formula (III). More specifically, a half life of degradation at 90°C (i.e., a time period required to break down polyperoxide by 50%) is a few minutes or shorter for the degradable polymer having the structure represented in General Formula (I), about one hour for the degradable polymer having the structure represented in General Formula (II), and 5 hours or longer for the degradable polymer having the structure represented in General Formula (III). As such, the degradation property is highly dependent on the structure.

As described above, the introduction of the substituents in the unsaturated group (diene group) of the diene monomer gives the degradable polymer a structure easy to break down. Alkyl group is preferably as the substituent.

A specific example is given in Table 1 which shows degradation properties of the following compounds: a compound ("polyperoxide-1" in Table 1) represented by General Formula (I) where R¹ is a methyl group, R² is a methyl group, R³ is a methyl group, and R⁴ is an ethoxy carbonyl group; a compound ("polyperoxide-2" in Table 1) represented by General Formula (II) where R⁵ is a methyl group, R⁶ is a methyl group, and R⁷ is an ethoxy carbonyl group; and a compound ("polyperoxide-3" in Table 1) represented by General Formula (III) where R⁸ is a methyl group, and R⁹ is a methoxy carbonyl group.

**Table 1**

| | Degradation Temp (°C) | Degradation Time (hours) | Degradation Ratio (%) |
|---|---|---|---|
| Polyperoxide-1 | 50 | 3 | 35.3 |
| | | 5 | 53.1 |
| | | 7 | 64.0 |
| | 70 | 0.5 | 53.2 |
| | | 1 | 80.5 |
| | 90 | 0.5 | 100 |
| Polyperoxide-2 | 90 | 1 | 45.8 |
| | | 3 | 88.1 |
| | | 5 | 97.8 |
| | 110 | 0.5 | 91.1 |
| Polyperoxide-3 | 90 | 3 | 34.5 |
| | | 5 | 45.5 |
| | 110 | 1 | 76.7 |
| | | 2 | 90.7 |

R¹, R², and R³ in General Formula (I), R⁵ and R⁶ in General Formula (II), and R⁸ in General Formula (III) are independently an alkyl group or an aromatic group. More specifically, the alkyl group is more preferably a straight or branched alkyl group of a carbon number of 1 to 18, such as methyl group, ethyl group, propyl group, butyl group. The aromatic group is more preferably an aromatic group of a carbon number of 6 to 10 such as phenyl group, naphthyl group, and the like. Moreover, n in General Formulae (I) to (III) is not particularly limited, but is preferably in a range of 2 to 1000.

In the present embodiment, a radical alternating copolymerization with a dienemonomer and oxygen is carried out to synthesize a copolymer having peroxide bonds in its main chain. The "radical alternating copolymerization with the dienemonomer and oxygen" is alternating copolymerization in which the diene monomer is bonded with oxygen. The "copolymer" is a polymer having such a structure that compounds to be polymerized (here, the diene monomer and oxygen) are alternatively repeated.

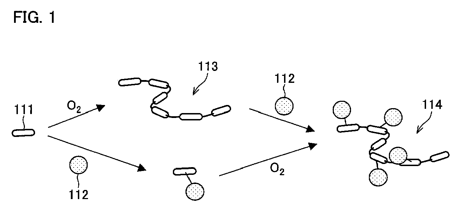

There are two methods to give the polyperoxides the function. The first one is the scheme illustrated in the lower portion of Fig. 1. A function-having group (function-having substituent) 112 (hereinafter, may be referred to as the substituent 112 simply) is introduced in a monomer 111. Then, the monomer 111 is copolymerized with oxygen thereby obtaining a polyperoxide 114 to which the function is given. The second one is the scheme illustrated in the upper portion of Fig. 1. The monomer 111 is copolymerized with oxygen thereby obtaining a polyperoxide 113. Then, the function-having group 112 is bonded to the polyperoxide 113, thereby obtaining the polyperoxide 114 to which the function is given.

One example of the function-having group introduction is for introducing a drug such as a medical drug for human body or the like. For example, a molecule of an anti-cancer agent, a polysaccharide, a pharmacologically active substituent, an oligopeptide, an oligonucleotide, or the like is introduced as the function-giving group.

Another example of the function-having group introduction is for giving water solubility. For example, a hydrophilic group such as hydroxyl group, carboxyl group, oligoethylene glycol, or the like is introduced as the function-giving group.

Still another group of the function-having group introduction is for giving biodegradability. For example, a molecule such as polylactic acid, polyglycolic acid or the like is introduced as the function-giving group.

Moreover, function-having groups of different properties may be introduced into the polyperoxide.

Examples of designing a novel degradable polymer material using such a polyperoxide encompass:
(1) introduction of a reactive group, that is, a function-having group into the polyperoxide thereby giving the polyperoxide the function;
(2) synthesis of a water-soluble polyperoxide for phase separation behavior; and
(3) synthesis of polyperoxide using a polylactic acid macromonomer.

The method (1) can be carried out by introducing the reactive group (function-having group) or by introducing a drug component. The former can be carried out by introducing the reactive group in the monomer and then synthesizing the polyperoxide from the reactive group-introduced monomer or by introducing the reactive group into the polyperoxide via a polymer reaction. The latter can be carried out by reacting the reactive group-introduced polyperoxide with the drug component, or by introducing the drug component in the monomer and then performing the polymerization with the drug component-introduced monomer.

The method (2) can be carried out by introducing a polar group or by causing a LCST type phase transition, in which the phase separation of the polymer is caused in response to a slight temperature change. The former involves introducing a carboxylic acid, hydroxyl group, or oligoethylene oxide in a side chain of the polyperoxide thereby to synthesize the water-soluble polyperoxide. The latter involves performing the LCST type phase transition of a polyperoxide aqueous solution and controlling the temperature for the phase transition.

The method (3) can be carried out by designing a PLA macromonomer or by designing a composite material. The former involves synthesis of a PLA macromonomer with an initiating end introduced therein, and synthesis of a PLA macromonomer with an terminating end introduced therein. The latter involves synthesis of a branched PLA polyperoxide and controlling degradation behavior of the branched PLA polyperoxide.

The applicant has developed polyperoxides by such radical alternating copolymerization with the diene monomer and oxygen. The polyperoxides are easily broken down by various stimuli such as heat, light, pH, enzyme, and the like, and have quite different degradation reaction mechanisms from the conventional degradable polymer. The polyperoxides developed by the applicant are readily broken down to lower molecular-weight compounds via a radical chain.

One possible practical application of the polyperoxide as a medical polymer material is to prepare a polyperoxide with a drug molecule in a side chain thereof in advance. The polyperoxide is carried down to a desired portion stably and broken down by a certain stimulus thereby releasing the drug molecule in an active form. To attain this, for example, the introduction of the reactive group into the polyperoxide may be carried out by introducing a highly reactive substituent such as an azide, isocyanate, or the like. Moreover, examples of the drug molecule encompass 5-fluorouracil and sugars.

Furthermore, to be used as a medical material, the polyperoxide may be used in a form of a water-soluble polymer.

Moreover, the LCST phenomenon (phenomenon in which a polymer causes phase separation in response to a slight temperature change) is an important phenomenon, which leads to development of a gel effective in the release of the drug.

Furthermore, the polyperoxide is easy to break down by various stimuli. Therefore, the polyperoxide may conjugated with a biocompatible polymer material and a bioabsorbable material, so that the polyperoxide will be stably existed until the degradation is required and at an *in vivo* location the degradation is required.

Moreover, the diene monomer may be a diene monomer containing a compound having at least two diene groups. This gives the degradable polymer a gel structure.

The gel (gel structure compound) has a 3-dimensional network structure, which gives the gel an excellent strength as well as insolubility in any solvent. Thus there is a big problem in disposing the gel. Especially, a gel derived from a vinyl polymer has a frame formed with carbon-carbon bonds and is poor in degradability. These problems can be solved by replacing these compounds with the degradable polymer according to the present embodiment, which has the gel structure having a polyperoxide at a cross linking point.

The gel is defined as a polymer having a 3-dimensional network structure insoluble in any solvent, and a swollen product of such a polymer. The gel is a material that has an intermediate state between solid and liquid. In a solvent, the gel absorbs the solvent thereby to swell in volume to a certain limit, but will not dissolve therein. Examples of its solid property are: gel absorbing the solvent therein can be brought up; and the gel can be deformed by applying a stress or by cutting. One example of its liquid property is that a low molecule can diffuse in the gel with a very large diffusion coefficient. Moreover, the gel is a open-system material, which can exchange energy, a material, or information by acting an outer environment. Further, it is known that the gel shows a phenomenon called "volume phase transition" in which the gel reversibly and non-continuously changes its volume by and according to a change (solvent composition, temperature, pH change) in its outer environment. Many studies have been conducted on functional gels such as stimulus responsive DDS, artificial muscle, sensor, shape-memory material, and the like.

Therefore, the use of the degradable polymer according to the present embodiment having the gel structure makes it possible to realize the functional gel with an excellent degradability.

The synthesis of the degradable polymer having the gel structure may be carried out by (i) forming a cross-linking structure concurrently with the polymerization, or by (ii) synthesizing a straight polymer and then cross-linking the straight polymer by a chemical reaction. More preferable synthesis of the degradable polymer having the gel structure is to synthesize a polymer that is a polymer (straight polymer) with polymerizing groups (diene groups) in its side chain or on both ends, or a macro monomer and then performing the method (i). In this way, a degradable polymer having the gel structure is produced by radical copolymerization with oxygen.

The synthesis of the polymer having diene groups on both the ends of the polymer chain can be carried out by living anion polymerization, but is not limited thereto. The synthesis of the polymer having diene groups on the both ends of the polymer chain can be carried out by another polymerization such as living cation polymerization, living coordinate polymerization, living radical polymerization, or living open-ring polymerization.

Moreover, apart from the living polymerization, any method that can introduce two or more diene groups in the polymer can be adopted to the synthesis of the polymer gel by alternating copolymerization with oxygen. For example, a polymer (such as polyethylene glycol) having hydroxyl groups on both ends is converted to alcoxide by adding n-butyl lithium thereto, and then mixed with sorbic chloride in the same way as in synthesis of MT (31) described later in Examples, thereby to introduce a diene group on each end of the polymer chain.

In the case of using a monomer, such as styrene, in which re-bonding is predominantly prevented in radical polymerization, a polymer is obtained by using a radial polymerization initiating agent having a functional group such as hydroxyl group, and then the polymer is reacted with sorbic chloride or sorbic isocyanate, thereby easily synthesizing a polymer having diene groups on both the ends of the polymer chain.

As described above, the degradable polymer (alternating copolymer) according to the present invention has the substituent such as a drug, hydrophilic group, biodegradable group or the like. With this arrangement, the degradable polymer according to the present invention is usable in the medical field and medical material field, is applicable to DDS and gene delivery system, and is adaptable to proving a novel polymer material and environmentally friendly material.

As described above, a degradable polymer according to the present invention is a degradable polymer having in its main chain a peroxide bond by radical alternating copolymerization of a diene monomer and oxygen, the degradable polymer having a drug molecule in its side chain.

Moreover, a degradable polymer according to the present invention is a degradable polymer having in its main chain a peroxide bond by radical alternating copolymerization of a diene monomer and oxygen, the degradable polymer having a water soluble substituent in its side chain.

Moreover, a degradable polymer according to the present invention is a degradable polymer having in its main chain a peroxide bond by radical alternating copolymerization of a diene monomer and oxygen, the degradable polymer having a biodegradable substituent in its side chain.

Moreover, a method of the present invention for producing any of the above degradable polymers having in its main chain a peroxide bond by radical alternating copolymerization of a diene monomer and oxygen, the method including: performing the radical alternating copolymerization after bonding the diene monomer with the substituent, so as to have the structure in which the degradable polymer includes the substituent in its side chain.

Moreover, a method of the present invention for producing any of the above degradable polymers having in its main chain a peroxide bond by radical alternating copolymerization of a diene monomer and oxygen, the method including: bonding the substituent with the degradable polymer in its side chain

In addition to any arrangement described above, a method according to the present invention having is arranged such that the substituent is at least one selected from the group consisting of a substituent made of a drug molecule, a water soluble substituent, and a biodegradable substituent.

### [EXAMPLES]

### [Example 1]

### (Introduction of Reactive Group into Polyperoxide to Provide Function)

It is known that oxygen works as a prohibiting agent in radical polymerization. However, alternating copolymerization with oxygen and vinyl monomer or diene monomer is possible under some polymerization conditions. Such alternating copolymerization gives a degradable polyperoxide having peroxide bonding in its main chain. A polyperoxide with a function group introduced therein can be produced easily by reacting highly reactive isocyanate with an alcohol having the function group. Thus, a polyperoxide was designed in such a manner that an isocyanate derivative was synthesized from sorbic acid, and then the isocyanate derivative was reacting with an alcohol having a functional group.

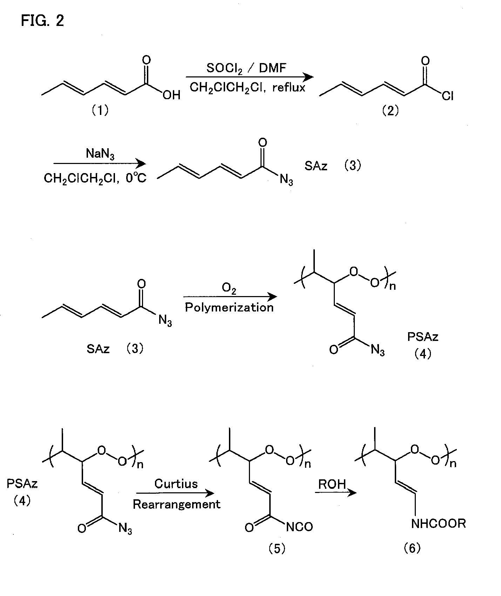

Following the reaction formula in the upper portion of Fig. 2, sorbic azide (SAz) (3) was synthesized from sorbic acid (1).

More specifically, 1, 2-dichloro ethane solution of 20ml in which sorbic acid of 5.6g (1) (50mmol) thionyl chloride of 4ml (55mmol), and N,N-dimethyl formamide (catalyst quantity) are contained was refluxed until production of hydrochloric acid was stopped. After the mixture was then cooled down to a room temperature, unreacted thionyl chloride and the solvent were removed under reduced pressure. The residue thus obtained was distilled under reduced pressure (0.5 torr, 40°C) thereby purifying sorbic chloride (2) with a yield of 92%.

Into dispersion of sodium azide of 3.9g (60mmol) in 1, 2-dichloroethane, the sorbic chloride (2) was dropped over about 30 minutes at 0°C under nitrogen gas stream. The mixture thus obtained was stirred overnight. Thereafter, 1.3g of sodium azide was added thereto. The mixture thus obtained was stirred overnight. After reaction, a produced sale was filtered out, and a filtrate was washed with ice water and then dried with sodium sulfuric anhydride. Then, the solvent was removed under reduced pressured. In this way, a sorbic azide (3) was obtained. The sorbic azide was unstable even at room temperature, no purification thereof was carried out. Therefore, the crude product was used as a monomer as it was. Yield of the crude produce was 63%. The sorbic azide (3) thus obtained was a liquid at room temperatures and its structure was identified by ¹H-NMR and ¹³C-NMR. Results of the measurements are as follows.

¹H-NMR (400 MHz, CDCl₃) δ 7.35 (dd, J = 9.6 Hz, and 15.2 Hz, CH=CHCO, 1H), 6.29 (m, CH₃CH=CH and CH₃CH=CH, 2H), 5.79 (d, J = 15.2 Hz, CH=CHCO, 1H), 1.90 (d, J = 5.6 Hz, CH₃CH=CH, 3H); ¹³C-NMR (100 MHz, CDCl₃) δ 171.6 (C=O), 146.7 (CH=CHCO), 141.9 (CH₃CH=CH), 129.4 (CH₃CH=CH), 119.7 (CH=CHCO), 18.5 (CH₃CH=CH)

Next, following the reaction formula in the middle portion of Fig. 2, polysorbic azide (PSAz) (4), which is a polyperoxide, was synthesized from sorbic azide (SAz) (3) by copolymerization.

The polymerization was carried out in the following manner. The monomer of 1g, azobis (4-methoxy-2,4-dimethylvaleronitrile) (AMVN) (low temperature initiator) of 20mg, and 1, 2-dichloroethane (solvent) of 20ml were introduced in a pear-shaped flask of 50ml. Then, the polymerization was carried out under ambient pressure at 30°C for 6 hours blowing oxygen therein. After the reaction, the polymerization solvent was poured in a large quantity of n-hexane, and a polymer was precipitated. The solvent was removed by decantation, and then re-precipitation was carried out twice in a mixed solvent of diethylether/n-hexane (= 1/1).

Then, following the reaction formula in the lower portion of Fig. 2, it was converted to isocyanate (5) by Curtius rearrangement. Next, using an alcohol having a function-having group (functional group) R, a polyperoxide (6) having the function-having group in its side chain was synthesized.

When an acid azide compound is left at room temperatures or heated slightly, Curtius rearrangement occurs, giving isocyanate. Isocyanate, which is highly reactive, reacts with an alcohol having a functional group thereby easily introducing the functional group in a polyperoxide without breaking down the polyperoxide.

The reaction was carried out in a dark room with polysorbic azide (PSAz) of 0.1g and an alcohol (ROH) having the functional group in 10 equivalent to PSAz, which were dissolved in a solvent. A conversion ratio from the acid azide to urethane was determined by ¹H-NMR.

NMR spectra before and after the reaction showed that a peak of methane group of PSAz was shifted from 6.98ppm to 6.80ppm, and peaks derived from 1-propanol appeared at 4.08, 1.26, 0.94ppm. This confirmed the production of urethane. Moreover, differential thermal analysis showed a large exothermic peak due to thermolysis of azide group from approximately 70°C. In the polymer after the reaction, the heat release value was reduced. This showed that the reaction was preceded.

Moreover, alcohols given in Fig. 3 were used apart from 1-propanol (7). The solvent was chloroform in (7) to (9), and THF (tetrahydrofrane) in (10) and (11). The reactions were carried out at 30°C for 24 hours. In (7) to (11), conversion ratios to copolymers were 28.2%, 26.9%, 27.1%, 22.3%, and 25.4%.

### [Example 2]

As described below, following the reaction illustrated in Fig. 4, a diene monomer (14) with 5-FU (5-fluorouracil) (12) introduced therein was copolymerized directly with oxygen, thereby to synthesize a branched 5-FU type polyperoxide (16). 5-FU was immobilized on the polymer via amide bonding and would be released by hydrolysis after decomposition of polyperoxide. If an isocyanate-type monomer was successfully obtained, urea bonding type polyperoxide containing 5-FU could have been synthesized successful.

Firstly, the 5-fluoroureacil sorbic amide (5-FUSA) (15) was synthesized as bellow. Into 15ml of hexamethyldisilazan (HMDS), 4.0g (0.03mol) of 5-fluoro uracil (12) and a catalyst quantity of trimethylchloride were added. Then the mixture was refluxed for 5 hours to obtain (13). The reaction was proceeded as ammonia was produced. After that, the resultant was cooled down and excess HMDS was removed therefrom under reduced pressure. The residue was dissolved in dry acetonitrile. Then, an acetonitrile solution in which 4.0g of sorbic chloride (14) was dissolved was dropped in the solution in an ice bath. After that, the reaction solution was stirred over night at room temperature. Then, the reaction was stopped by adding methanol therein. After the solvent was removed therefrom under reduced pressure, a precipitated target material was recrystallized in acetone. In this way, the 5-FUSA (15) was obtained with a yield of 36.0%.

The resultant 5-FUSA (15) had a melting point of 170°C to 171°C and was in a power form. Results of analysis of the 5-FUSA (15) with ¹H-NMR and ¹³C-NMR are shown below.

¹H-NMR (400 MHz, CD₃OD) δ 8.29 (d, J = 7.2 Hz, CH=CFCO, 1H), 7.54 (m, CH=CHCO, 1H), 7.07 (d, J = 15.2 Hz, CH=CHCO, 1H), 6.43 (m, CH₃CH=CH, 2H), 1.91 (d, J = 4.8 Hz, CH₃CH=CH, 3H); ¹³C-NMR (100 MHz, CD₃OD) δ 166.3 (CH=CHCO), 159.4 (d, J = 27.2 Hz, CH=CFCO), 150.0 (NCONH), 149.6 (CH=CHCO), 144.1 (d, J = 237 Hz, CH=CFCO), 143.9 (CH₃CH=CH), 131.6 (CH₃CH=CH), 124.0 (d, J = 37.1 Hz, CH=CFCO), 121.4 (CH=CHCO), 19.0 (CH₃CH=CH)

Then, the 5-FUSA (15) and oxygen were copolymerized in the following manner. The monomer of 1g, azobis (4-methoxy-2,4-dimethylvaleronitrile) (AMVN) (low temperature initiator) of 20mg, and tetrahydrofrane (solvent) of 6g were introduced in a pear-shaped flask of 50ml. Then, the polymerization was carried out under ambient pressure at 30°C for 6 hours blowing oxygen therein. After the reaction, the polymerization solvent was poured in a large quantity of n-hexane, and a polymer was precipitated. The solvent was removed by decantation, and then re-precipitation was carried out three times in a mixed solvent of acetone/n-hexane (= 1/10). In this way, a branched 5-FU type polyperoxide (16) was obtained with a yield of 19.8%.

### [Example 3]

Sugars are compounds most abundant in the nature. In living bodies, sugars are not only energy source, but also play a role for recognizing of various biomaterials by bonding with proteins or surfaces of cells. Many studies have been conducted on drug delivery in which bonding with sugar is utilized. Therefore, synthesis of a polyperoxide having a sugar in its side chain was conducted here. In order to avoid problems in solubility and isolation production, a sugar protected with acetyl group was used. It was found that a polyperoxide was obtained as expected, and the polyperoxide was easy to break down like the other derivatives. It is expected that a sugar of more complex structure can be introduced similarly.

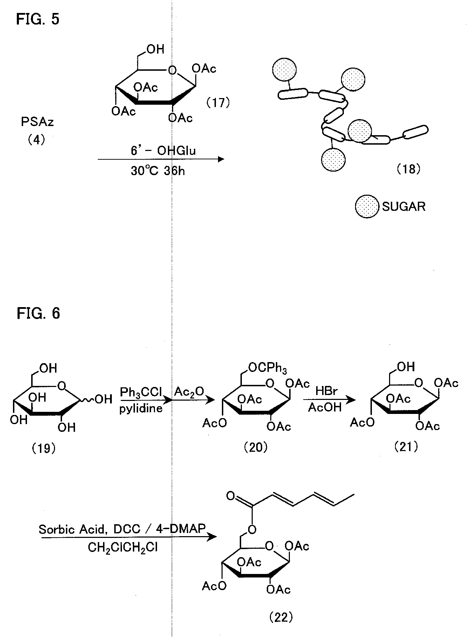

Following the formula illustrated in Fig. 5, a compound (18) was obtained in which 1,2,3,4-tetraacetyl-6-hydroxyglucose (6'-OHGlu) (17) was introduced in PSAz. According to NMR, a ratio of introduction was 24.2%.

Moreover, following the formula illustrated in Fig. 6, a compound (22) was obtained by bonding sorbic acid with 1,2,3,4-tetraacetyl-6-hydroxyglucose (6'-OHGlu) (21). Then, the compound (22) was copolymerized with oxygen under the same conditions as the copolymerization of SAz was carried out.

That is, under a stream of nitrogen, 6.8g (0.024mol) of triphenylmethylchloride was added in a pyridine solution in which D-(+)-glucose of 5.4g (0.03mol) was contained. Then, the solution thus obtained was stirred for 24 hours at a room temperature. After 22g (0.21mol) of acetic anhydride was then dropped therein over 30min, the solution was further stirred for 6 hours. After the reaction, the solvent and excel acetic anhydride were removed under reduced pressure, and the residue thus obtained was dissolved in chloroform, thereby obtained a chloroform solution. The chloroform solution was neutralized with sodium carbonate and then washed with distilled water and then saturated salt water. Then, the chloroform solution was dried with sodium sulfuric anhydride. After it was concentrated under reduced pressure, the resultant was crystallized in hexane, and recrystallized in diethylether/n-hexane thereby obtaining 1,2,3,4-tetraacetyl-6-triphenylmethylglucose with a yield of 40.4%.

Into 30ml of acetic acid, 3.4g (5.8mmol) of 1,2,3,4-tetraacetyl-6-triphenylmethylglucose was dissolved. Into the solution thus obtained, 2ml of hydrobromic acid (48%) was dropped while the solution was kept at temperatures in a range 10°C to 15°C. The dropping immediately changed a color of the solution to yellow. Shaking the solution for about 1 minutes precipitated triphenylmethylbromide, which was then filtered out. A filtrate was poured into 200ml of ice water, thereby precipitating white solid, which was then extracted with chloroform (50ml X 4 times). The chloroform solution thus obtained was washed with water a few times, and dried with sodium sulfuric anhydride. The solution thus obtained was concentrated under reduced pressure, thereby obtaining 1,2,3,4-acetyl-6-hydroxyglucose (21), which was then recrystallized with diethylether/n-hexane. In this way, 1,2,3,4-acetyl-6hydroxyglucose (21) was obtained with a yield of 73.8%.

Into an ice-bathed dichloroethane solution in which 2.0g (5.7mmol) of 1,2,3,4-tetraacetyl-6-triphenylmethylglucose (21), 0.64g (5.7mmol) of sorbic acid, 0.35g of 4-dimethylaminopyridine (4-DMAP) were dissolved, a dichloroethane solution in which dicyclohexyl imide (DCC) of 1.2g (5.8mmol) was contained was dropped in. After the dropping, the reaction was brought back to a room temperature and stirred overnight, thereby precipitating dicyclohexyl urea, which was then filtered out. A filtrate was concentrated under reduced pressure. Then, the concentrated was subjected to silica gel column chromatography (carrier; chloroform/ethyl acetate =10:1) to isolate a target product, 1,2,3,4-tetraacetyl-6-sorbic ester glucose (6-S-Glu) (22), which was then recrystallized in diethylether. This condensation reaction had a yield of 61.5%, and overall yield of the whole reaction was 18.4%. By using ¹H-NMR and ¹³C-NMR, a structure of 1,2,3,4-tetraacetyl-6-sorbic ester glucose (6-S-Glu) (22) was confirmed.

¹H-NMR (400 MHz, CDCl₃) δ 7.31 (m, CH=CHCO, 1H), 6.20 (m, CH₃CH=CH, 2H), 5.81 (d, J = 14.8 Hz, CH=CHCO, 1H), 5.74 (d, J = 8.4 Hz, C1(H), 1H), 5.28 (t, J = 9.6 Hz, C3(H), 1H), 5.17 (m, C2(H) and C4(H), 2H), 4.31 (m, C6(H₂), 2H), 3.91 (m, C5(H), 1H), 2.12-2.02 (s, CH₃CO, 12H), 1.87 (d, J = 5.2 Hz, CH₃CH=CH, 3H); ¹³C-NMR (100 MHz, CDCl₃) δ 170.1, 169.3, 169.2, 168.9 (CH₃CO), 166.6 (CH=CHCO), 146.1 (CH=CHCO), 140.2 (CH₃CH=CH), 129.7 (CH₃CH=CH), 117.8 (CH=CHCO), 91.6 (C1), 72.8 (C3), 72.7 (C5), 70.1 (C2), 67.9 (C4), 61.4 (C6), 20.8-20.4 (CH₃CO), 18.7 (CH₃CH=CH)

The polymerization was carried out with a yield of 20.1% in the same manner as the polymerization of SAz was carried out.

### [Example 4]

For example, it is possible to prepare a polymer having a non-degradable polymer portion and a degradable polymer portion by introducing a polymer reactive substituent in a side chain of a polymer by polymerization to prepare the main chain of the polyperoxide and then to prepare the polymerizable group. In the present Example, a composite of the polymers having different degradabilities was formed thereby synthesizing a polymer material having a different property from those of the polymers used solely.

Firstly, 2-methacryloyloxyethyl sorbate (HEMAS) was synthesized. A 1,2-dichloroethane solution of 25ml (containing 5.6g (50mmol) sorbic acid, 4ml (55mmol) of thyonyl chloride, and 0.5g (catalyst quantity) of N,N-dimethylformamide) was refluxed for about 30min thereby to synthesize sorbic chloride, which was used in a following reaction as it was, without isolation or purification. Into an ice-bathed 1,2-dichloroethane solution containing 4.0g (50mmol) of pyridine and 6.5g (50mmol) of 2-hydroxyethylmethacrylate, a 1,2-dichloroethane solution containing sorbic chloride of 6.5g (50mmol) was dropped. After being stirred overnight at room temperatures, precipitated pyridine hydrochloride was filtered out from the solution with suction. A filtrate was washed with sodium hydrogen carbonate, distilled water, and salt water. An organic layer thus obtained was dried with sodium sulfuric anhydride. The resultant was subjected to silica gel column chromatography (carrier; ethyl acetate:n-hexane=2:1) thereby to obtain targeted HEMAS with a yield of 56.8%. The HEMAS was a liquid at an ordinary temperature and its structure was confirmed with ¹H-NMR and ¹³C-NMR.

¹H -NMR (400 MHz, CDCl₃) δ 7.28 (dd, J = 10.8 and 15.2 Hz, CH=CHCO, 1H), 6.21-6.14 (m, CH₃CH=CH and, CCO(CH₃)=CH₂, 3H), 5.79 (d, J = 15.6 Hz, CH=CHCO, 1H), 5.59 (s, CCO(CH₃)=CH₂, 1H), 4.41-4.38 (m, CH₂CH₂, 4H), 1.95 (t, J = 0.8Hz, COC(CH₃)=CH₂, 3H), 1.86 (d, J = 5.6 Hz, CH₃CH=CH, 3H); ¹³C-NMR (100 MHz, CDCl₃) δ 166.9 and 166.7 (C=O), 145.5 (CH=CHCO), 139.6 (CH₃CH=CH), 135.7 (COC(CH₃)=CH₂), 129.5 (CH₃CH=CH), 125.8 (COC(CH₃)=CH₂), 118.0 (CH=CHCO), 62.3 (CH₂CH₂), 61.7 (CH₂CH₂), 18.4 (COC(CH₃)=CH₂), 18.0 (CH₃CH=CH)

Next, a polyperoxide having methacryloyl group in its side chain was synthesized by copolymerization of HEMAS and oxygen at 30°C for 6 hours using AMVN as an initiator. As a result, a polymer having a cross-linked gel structure was obtained with a yield of 39.6%. The polymer thus obtained was insoluble in solvents.

### [Example 5]

On the other hand, polymerization is an effective method to synthesize a polyperoxide having methacryloyl group in its side chain but not having a cross-linked structure.

Firstly, PSAz was synthesized as described in the above Example. Then, PSAz was reacted with 2-hydroxyethylmethacrylate (HEMA) at 30°C for 24 hours thereby introducing methacryloyl groups in the side chains of PSAz. The resultant polymer showed ¹H-NMR spectrum in which a peak derived from hydrogen of methine group was at 6.8ppm, peaks derived from hydrogen of olefin of methacryloyl group were at 5.6ppm and 6.2ppm, and a peak derived from hydrogen of methyl group was at 1.9ppm. A substitution ratio from azide group to methacryloyl group in the side chain was 29.9%. Moreover, the resultant polymer obtained by the reaction has a number average molecular weight of 3.0 x 10³. It was confirmed that no degradation of peroxide chain occurred.

The polyperoxide thus obtained which had methacryloyl group in its side chain was subjected to radical polymerization. In order not to cleave the peroxide bonds, AMVN was used as an initiator. In this way, the polyperoxide thus obtained which had methacryloyl group in its side chain was copolymerized with 2-ethyl hexyl methaycrylate (HEMA).

The resultant polymer was insoluble in any solvent. This confirmed that the polyperoxide chain was not broken down under this condition and had a gel structure. Moreover, the resultant polymer was swollen with solvents such as methanol, toluene, THF, DMF, and the other. The polymer showed a DTA curve with an exothermic peak at 107.2°C due to thermolysis of azide group remained in the polymer and an exothermic peak at 128.2°C due to breaking of peroxide bonding.

Moreover, the polymer was refluxed in toluene for 1 hour. This gave the polymer solubility in solvent. The polymer after 1-hour reflux showed a DTA curve without an exothermic peak or weight reduction that is caused by the breaking of the peroxide bonding, which starts from approximately 100°C. This confirmed that the 1-hour reflux broken down the polymer and deprived the gel structure therefrom.

### [Example 6]

### (Synthesis of Water-soluble Polyperoxide and Phase Separation Behavior)

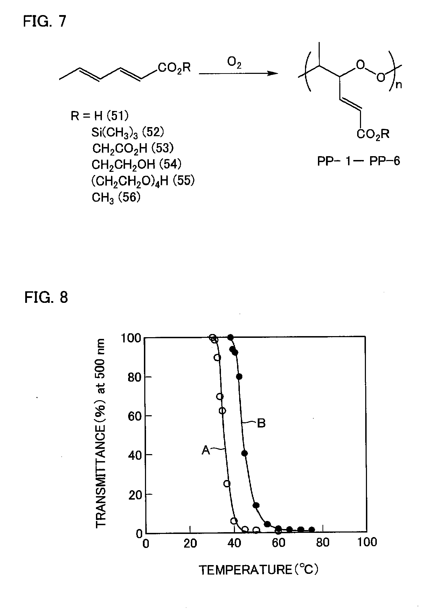

As illustrated in Fig. 7, a water-soluble polyperoxide was synthesized by introducing a hydrophilic group in its side chain, thereby controlling phase separation phenomenon of the polyperoxide. In order to introduce the hydrophilic group in the side chain of polyperoxide, monomers given in the left side of Fig. 7 were synthesized and copolymerized with oxygen. The following describes how the monomers were synthesized.

### <Synthesis of trimethylsilylsobate (Monomer having a Substituent (52))>

Dissolved in 100ml of 1,2-dichloroethane were 2.8g (25mmol) of sorbic acid and 2.55g (12.5mmol) of N,N'-bis(trimethylsilyl)urea. Then, the solution thus prepared was stirred at 40°C for 3 hours. After the reaction, the solvent was removed. Then, the solution was distilled under reduced pressure (2mmHG, 100°C) thereby obtaining trimethylsilylsorbate with a yield of 26%. Trimethylsilylsorbate is a liquid at an ordinary temperature. NMR spectrum data thereof is as follows:

¹H NMR (400 MHz, CDCl₃) δ 7.20 (dd, J = 10.4 and 15.2 Hz, CH=CHCO, 1H), 6.09-6.23 (m, CH₃CH=CH, 2H), 5.74 (d, J = 15.2 Hz, CH=CHCO, 1H), 1.85 (d, J = 5.2, CH₃CH=CH, 3H), 0.31 (s, Si(CH₃)₃, 9H)

### <Synthesis of Carboxymethylsorbate (Monomer having a Substituent (53))>

By reacting 6.5g of sorbic chloride and 5.0g of glycolic acid (30°C, 20 hours), carboxymethyl sorbate with a melting point in a range of 108°C to 109°C was obtained in a form of powder with a yield of 62.3%. NMR spectrum data thereof is as follows:

¹H NMR (400 MHz, CDCl₃) δ7.32-7.38 (m, CH=CHCO, 1H), 6.16-6.26 (m, CH₃CH=CH, 2H), 5.85 (d, J = 15.2 Hz, CH=CHCO, 1H), 4.73 (s, OCH₂CO, 2H), 1.87 (d, J = 5.2 Hz, CH₃CH=CH, 3H); ¹³C NMR (100 MHz, CDCl₃) δ173.22 (CO₂CH₂CO₂H), 166.36 (CO₂CH₂CO₂H), 146.96 (CH=CHCO), 140.78 (CH₃CH=CH), 129.64 (CH₃CH=CH), 117.13 (CH=CHCO), 60.02 (CO₂CH₂CO₂H), 18.72 (CH₃CH=CH)

### <Synthesis of 2-hydroxyethylsorbate (Monomer having a Substituent (54))>

Into an ice-bathed 1,2-dichloroethane solution (25ml) containing 5.6g (50mmol) of sorbic acid, 1.71g(50mmol) of ethyleneglycol, and 0.56g (5mmol) of 4-(dimethylamino)pyridine, dropped gradually was a 1,2-dichloroethane solution (20ml) containing 10.3g of dicyclohexylcarbodimide (DCC). After one-hour stirring in the ice bath, the solution thus prepared was stirred at a room temperature overnight. The resultant reaction solution was washed with 0.5M hydrochloric acid, saturated sodium hydrogen carbonate aqueous solution, and then saturated salt water. An organic phase thus obtained was dried with sodium sulfuric anhydride and purified by silica gel column chromatography (carrier; chloroform:tert-butylmethylether =1:1) with a yield of 53.8%. The resultant 2-hydroxyethylsorbate was a liquid at an ordinary temperature. Its structure was confirmed with ¹H-NMR and ¹³C-NMR, whose data is shown below.

¹H NMR (400 MHz, CDCl₃) δ7.30 (dd, J = 15.2 and 10.0 Hz, CH=CHCO, 1H), 6.16-6.25 (m, CH₃CH=CH, 2H), 5.81 (d, J = 15.2 Hz, CH=CHCO, 1H), 4.28-4.30 (m, CO₂CH₂CH₂OH, 2H), 3.85-3.88 (m, CO₂CH₂CH₂OH, 2H), 2.13 (t, J = 6.0 Hz, CO₂CH₂CH₂OH, 1H), 1.87 (d, J = 5.2 Hz, CH₃CH=CH, 3H); ¹³C NMR (100 MHz, CDCls) &167.61 (CO₂CH₂CO₂H), 145.77 (CH=CHCO), 139.98 (CH₃CH=CH), 129.61 (CH₃CH=CH), 118.17 (CH=CHCO), 66.00 (CO₂CH₂CH₂OH), 61.18 (CO₂CH₂CH₂CH), 18.63 (CH₃CH=CH)

### <Synthesis of Tetraethyleneglycol Monosorbic Acid Ester (Monomer having a Substitute (55))>

In the same manner as the synthesis of 2-hydroxyethyl sorbate, tetraethyleneglycol monosorbic acid ester was synthesized via condensation reaction with sorbic acid and tetraethyleneglycol. Purification was carried out with column chromatography (carrier: tert-butylmethylether). Its yield was 36.6%. The thus obtained tetraethyleneglycol monosorbic acid ester was a liquid at an ordinary temperature. Its structure was confirmed with ¹H-NMR and ¹³C-NMR, whose data is shown below.

¹H NMR (400 MHz, CDCl₃) δ7.28 (dd, J = 15.2 and 10.0 Hz, CH=CHCO, 1H), 6.11-6.23 (m, CH₃CH=CH, 2H), 5.81 (d, J = 15.2 Hz, CH=CHCO, 1H), 3.72-3.75 (m, OCH₂(CH₂OCH₂)₃CH₂OH, 2H), 3.68 (s, OCH₂(CH₂OCH₂)₃CH₂OH, 12H), 3.60-3.63 (m, OCH₂(CH₂OCH₂)₃CH₂OH, 2H), 2.65 (broad, OCH₂(CH₂OCH₂)₃CH₂OH, 1H), 1.86 (d, J = 5.2 Hz, CH₃CH=CH, 3H)

The polymerization of the monomers were carried out with each monomer above, an initiator (AMVN), and 1,2-dichloroethane (solvent) at 30°C for 6 hours blowing oxygen therein.

Moreover, in Fig. 7, 6 types of polyperoxides (PP) were prepared with different substituents (51) to (56). The 6 types of polyperoxides (PP) are referred to as PP-1 to PP-6 respectively in association with the substituents (51) to (56).

As a result of stirring trimethylsilylester type polymer PP-2 in methanol, hydrolysis reaction undertakes substantially quantitatively, thereby obtaining an alternating copolymer of sorbic acid and oxygen. The introduction of hydrophilic group gave all the polymers solubility in methanol, but gave only PP-5 solubility in water.

It was found that heating of an aqueous solution of PP-5 causes clouding from approximately 90°C, leading to phase separation. Such behavior has been confirmed in some polymers such as polyacrylamide or polyether. The temperature at which the phase separation occurs is called Lower Limit Critical Solution Temperature (LLCST). This has been intensively studied especially in the field of poly N-isopropylacrylamide and a gel thereof. Applications thereof to medical materials and DDS materials have been researched. However, the high phase separation temperature of PP-5 is at the same level as the degradation temperature of polyperoxide. This makes it difficult to evaluate PP-5 and is also a problem in practical application thereof. This example tried to develop a polymer in which the phase separation occurs around room temperatures, by lowering the phase separation temperature by copolymerization with a hydrophobic monomer.

Fig. 8 illustrates a phase diagram of the phase separation of 0.5wt% aqueous solution of the copolymer. Here, PP-5/6-75 is a polyperoxide obtained from a mixture of the monomer having the substituent (55) (the material of PP-5) and the monomer having the substituent (56) (the material of PP-6) in a weight ratio of 75:25. Similarly, PP-5/6-80 is a polyperoxide obtained from a mixture of the monomers (55) and (56) in a weight ratio of 80:20. In Fig. 8, A is the PP-5/6-75 and B is the PP-5 / 6-80.

Assuming that the phase separation point (LCST) is a temperature of the polyperoxide with a transparency of 50%, the LCST of the PP-5/6-75 and that of the PP-5/6-80 are different from each other by approximately 15°C. It was found that it is possible to control the LCST by a compositional change to alter the polymer in the hydrophilic property.

### [Example 7]

### (Synthesis of Polyperoxide from Polylactic Acid Macromonomer)

Via alternating copolymerization with oxygen, a diene monomer produces polyperoxide degradable by heat, light, redox, and enzyme. On the other hand, polylactic acid (PLA) is one recyclable material, which is produced from natural raw materials and hydrolyzable to lactic acid metabolically absorbable *in vivo.* PLA and polyperoxide are different in degradation property. It is expected to obtain a new function as a result of combination of polyperoxide and PLA.

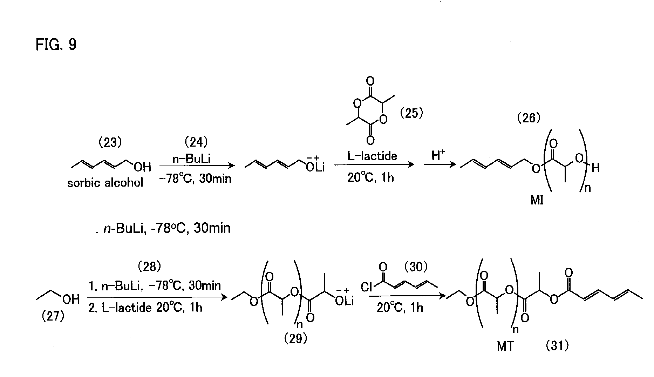

Firstly, as illustrated in Fig. 9, macromonomers MI (26) and MT (31) were prepared, in which dienyl group was introduced at a terminal of PLA, and which are formally called respectively Sorbic alcohol-initiated poly(lactic acid)macromonomer and Sorbic acid-terminated poly(lactic acid)macromonomer. Next, as illustrated in Figs. 10 and 11, radical polymerization with oxygen was carried out to prepare graft polymer PMI (32) and PMT (33), whose main chains have polyperoxide structures with PLA in their side chains.

More specifically, as illustrated in the upper portion of Fig. 9, the MI (26) as a polylactic macromonomer was synthesized from n-butyllithium (24) and sorbic alcohol (23) as starting materials by an effect of L-lactide (25).

The MI was synthesized in the following manner. That is, 1.25ml of n-butyllithium/n-hexane solution (1.6M) was added into a THF solution of sorbic alcohol via a Schlenk tube of 30ml under nitrogen gas atmosphere at -78°C. Then, the resultant solution was stirred for 30min. After that, 8ml (10mmol) of a THF solution (1.25M) of L-lactide was added therein. Polymerization temperature was quickly reduced to a room temperature by water bath. Then, the resultant solution was stirred for 1 hour. Then, the polymerization was stopped by adding a small quantity of acetic acid therein. Then, the solution was poured to a large quantity of a precipitating agent (diethylether:n-hexane = 6:4), thereby precipitating the polylactic acid macromonomer (MI), whose structure was then confirmed with ¹H-NMR and ¹³C-NMR. Data of the ¹H-NMR and ¹³C-NMR measurements are shown below.

¹H NMR (400 MHz, CDCl₃) δ6.25 (dd, J = 15.2 and 10.4 Hz, CH=CHCH₂), 6.05 (dd, J = 15.2 and 10.4 Hz, CH₃CH=CH), 5.77 (dq, J = 15.2 and 6.8 Hz, CH₃CH=CH), 5.58 (dt, J = 15.2 and 6.8 Hz, CH=CHCH₂), 5.16 (q, J = 6.8 Hz, CHCH₃ of PLA), 4.62 (t, J = 6.0 Hz, CH=CHCH₂), 4.36 (q, J = 7.2 Hz, terminal-CHCH₃), 1.77 (d, J = 6.8 Hz, CH₃CH=CH), 1.58 (d, J = 7.2 Hz, CHCH₃ of PLA); ¹³C NMR (100 MHz, CDCl₃) δ169.61 (C=O), 68.97 (CHCH₃), 16.68 (CHCH₃)

The sorbic acid derivative per se undertakes anion polymerization and thus cannot be directly added in the starting reaction. Therefore, as illustrated in the lower portion of Fig. 9, the MT (31) as a polylactic macromonomer was synthesized from n-butyllithium (28) and ethanol (27) as starting materials. In the synthesis of the MT (31), a PLA living anion (29) was terminated with sorbic chloride (30).

The MT was synthesized in the following manner. That is, 0.63ml of n-butyllithium/n-hexane solution (1.6M) was added into a THF solution (5ml) of dried ethanol (0.05g) via a Schlenk tube of 30ml under nitrogen gas atmosphere at -78°C. Then, the resultant solution was stirred for 30min. After that, 8ml (10mmol) of a THF solution (1.25M) of L-lactide was added therein. Polymerization temperature was quickly reduced to a room temperature by water bath. Then, the resultant solution was stirred for 1 hour. Then, the polymerization was stopped by adding 1ml (1.2mmol) of a THF solution of sorbic chloride at -78°C and then stirring it for one hour. Then, the solution was poured to a large quantity of a precipitating agent (diethylether:n-hexane = 6:4), thereby precipitating the polylactic acid macromonomer (MT), whose structure was then confirmed with ¹H-NMR and ¹³C-NMR. Data of the ¹H-NMR and ¹³C-NMR measurements are shown below.

¹H NMR (400 MHz, CDCl₃) δ7.22 (m, COCH=CH), 6.18 (m, CH₃CH=CHCH=CH), 5.83 (d, J = 15.2 Hz, COCH=CH), 5.16 (q, J = 6.8 Hz, CHCH₃ of PLA), 4.19 (q, J = 6.4 Hz, CH₃CH₂O), 1.85 (d, J = 4.8 Hz, CH₃CH=CH), 1.58 (d, J = 6.8 Hz, CHCH₃ of PLA), 1.27 (t, J = 6.8 Hz, CH₃CH₂O); ¹³C NMR (100 MHz, CDCl₃) δ169.76 (C=O), 69.13 (CHCH₃), 16.77 (CHCH₃)

In this way, the macromonomers (MI and MT) of two types with different terminal groups were obtained. A molecular weight change in the MI (26) against the quantity of the initiating agent is shown in Table 2. A molecular weight change in the MT (31) against the quantity of the initiating agent is shown in Table 3. Tables 2 and 3 confirmed that the molecular weights of these monomers are controllable with the quantity of the initiating agents.

**Table 2**

| [L-Lactide]/[n-BuLi] (mol/mol) | PLA Production Yield (%) | Number Average Molecular Weight (Mn) | Polydispersity (Mw/Mn) |
|---|---|---|---|
| 50 | 94.4 | 6.3 | 1.7 |
| 25 | 85.6 | 5.0 | 1.7 |
| 10 | 57.4 | 3.1 | 1.4 |
| 5 | 37.8 | 2.5 | 1.2 |

**Table 3**

| [L-Lactide]/[n-BuLi] (mol/mol) | PLA Production Yield (%) | Number Average Molecular Weight (Mn) | Polydispersity (Mw/Mn) |
|---|---|---|---|
| 25 | 94.2 | 5.0 | 1.7 |
| 10 | 52.5 | 3.3 | 1.3 |
| 5 | 36.1 | 2.8 | 1.2 |

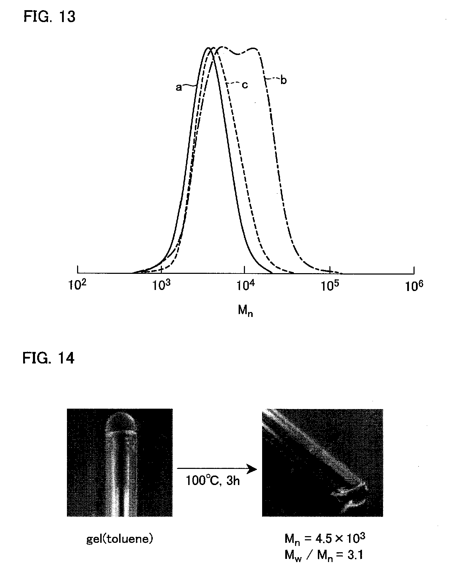

It was confirmed that the copolymerization of the MI (26) with oxygen as illustrated in Fig. 10 gave a higher molecular weight to the synthesis of a polyperoxide PMI (32). Further, it was confirmed that the heating of the resultant polyperoxide reduced the molecular weight due to the cleavage of the peroxide bonding. Fig. 13 illustrates a change in molecular weight distribution of the MI (26) (curve (a) in Fig. 13), the PMI (32) obtained by the copolymerization of the MI (26) with oxygen (curve (b) in Fig. 13), and the PMI (32) after thermolysis (110°C, 5 hours) (curve (c) in Fig. 13). The molecular weight distribution was measured by GPC.

As illustrated in Fig. 11, similar polymerization was performed with the MT (31), thereby obtaining a polyperoxide PMT (33).

The copolymerization was carried out with the monomer of a predetermined quantity (normally, 0.5g), azobis(4-methoxy-2,4-dimethylvaleronitrile) (AMVN) (low temperature initiator) and a solvent in a pear-shaped flask of 50ml blowing oxygen therein in a thermostatic chamber. The polymerization mixture obtained by the reaction was poured to a large quantity of a precipitating agent (diethyl ether: n-hexane = 6:4), thereby precipitating the polymer, which was then collected via filtration. In the molecular weight measurement, a peak of a macromonomer and a peak of PP obtained from the macromonomer cannot be sufficiently separated to perform quantitative analysis thereon using only an RI detector. Thus, by utilizing that the terminal dienyl group of the macromonomer absorbs UV (ultraviolet light) of a wavelength of 254nm, quantitative comparison of elution curves obtained by using an RI (differential refractive index) and a UV detector before and after the polymerization was performed so as to calculate a reaction ratio of dienyl group. This reaction ratio of dienyl group was regarded as a polymerization ratio of the copolymerization of the PLA macro monomer and oxygen.

As described above, the two types of polylactic macromonomers were synthesized by ring-opening anion polymerization of L-lactide by modifying an initiating end or a propagating end with dienyl group. The resultant macromonomers were controllable in molecular weights by the quantity of the initiators. In this Example, the resultant macromonomers were copolymerized with oxygen thereby to synthesize polyperoxides, and molecular weights of the polyperoxides were confirmed. Further, molecular weight reductions of the polyperoxides due to thermolysis were confirmed. Comparison of polymerization reactivities of the two macromonomers showed that MT had a higher copolymerization rate than MI, indicating that MT had a higher reactivity than MI. However, MT showed a smaller change in molecular weight than MI.

As described above, the two types of polylactic macromonomers were successfully synthesized by ring-opening anion polymerization of L-lactide by modifying an initiating end or a propagating end with dienyl group. Copolymeriztion of the resultant macromonomers with oxygen produced the polyperoxides successfully.

Needless to say, combination of the synthesis methods of the MI (26) and MT (31) illustrated in Fig. 9 makes it possible to synthesize a telechelic polylactic acid having a diene group at each of the initiating end and terminating end.

More specifically, the PLA living anion is obtained from n-butyllithium (24) and sorbic alcohol (23) as starting materials by the effect of L-lactide (25). Here, such polymerization termination by adding acetic acid, and precipitation of the polylactic acid macromonomer by adding the polymerization solution to a large amount of precipitating agent is not performed. Instead, the polymerization termination is performed in the same manner as in the synthesis of the MT (31), that is, the termination of the PLA living anion by using sorbic chloride (30). This makes it possible to synthesize the telechelic polylactic acid having a diene group at each of the initiating end and terminating end. The initiating end of the telechelic polylactic acid has a structure identical with the end of MI (26) and the terminating end of the telechelic polylactic acid has a structure identical with the end of MT (31).

The telechelic polylactic acid was synthesized in the following manner. That is, 0.63ml of n-butyllithium/n-hexane solution (1.6M) was added into a THF solution of sorbic alcohol via a Schlenk tube of 30ml under nitrogen gas atmosphere at -78°C. Then, the resultant solution was stirred for 30min. After that, 8ml (10mmol) of a THF solution (1.25M) of L-lactide was added therein. Polymerization temperature was quickly reduced to a room temperature by water bath. Then, the resultant solution was stirred for 1 hour. Then, the polymerization was stopped by adding 1ml (1.2mmol) of a 1.2 M THF solution of sorbic chloride at -78°C and then stirring it for one hour. Then, the solution was poured to a large quantity of a precipitating agent (diethylether:n-hexane = 6:4), thereby precipitating the telechelic polylactic acid, whose structure was then confirmed with ¹H-NMR and ¹³C-NMR. Data of the ¹H-NMR and ¹³C-NMR measurements are shown below.

¹H NMR (400 MHz, CDCl₃) 7.27 (m, COCH=CH), 6.17-6.28 (m, CH=CHCH₂O, COCH=CHCH=CH), 6.04 (dd, J = 15.2 and 12.0 Hz, CHCH=CHCH₂O), 5.74-5.85 (m, CH=CHCH₂O, COCH=CH), 5.58 (dt, J = 15.2 and 7.6 Hz, CH=CHCH=CHCH₂), 4.62 (t, J = 6.0 Hz, CH=CHCH₂), 4.36 (q, J = 6.8 Hz, terminal-CHCH₃), 1.86 (d, J = 5.2 Hz, CH₃CH=CHCH=CHCO), 1.77 (d, J = 6.8 Hz, CH₃CH=CHCH=CHCH₂), 1.58 (d, J = 6.8 Hz, CHCH₃ of PLA); ¹³C NMR (100 MHz, CDCl₃) 169.92 (C=O), 69.30 (CHCH₃), 16.94 (CHCH₃)

The molecular weight of the telechelic polylactic acid was controllable with the quantity of the initiating agent as in the synthesis of the MI and MT.

Furthermore, copolymerization of the telechelic polylactic acid with oxygen was carried out in the same manner as in the polymerization of MI or MT, thereby to synthesize a degradable polymer having a poly lactic structure. The degradable polymer was confirmed that it had a gel structure. Heat treatment (100°C, 3 hours) of the resultant degradable polymer as illustrated in Fig. 14 caused degradation due to cleavage of the peroxide bonds, and gave the degradable polymer solubility in solvents.

### [Example 8]

### (Synthesis of Polymer Gel by Alternating Copolymerization of Oxygen and Polymer having Diene Group)

As described in Example 7, it is possible to synthesize a gel-structured degradable polymer by the radical alternating copolymerization with oxygen and a polymer having a diene group at each end of its polymer chain.

In the present Example, a degradable polymer having a gel structure was synthesized by a method other than the living polymerization. More specifically, a polymer having a diene group on each end of its polymer chain was synthesized by performing polymerization with a radical polymerization initiating agent having a functional group such as hydroxyl group. This method is described below more specifically.

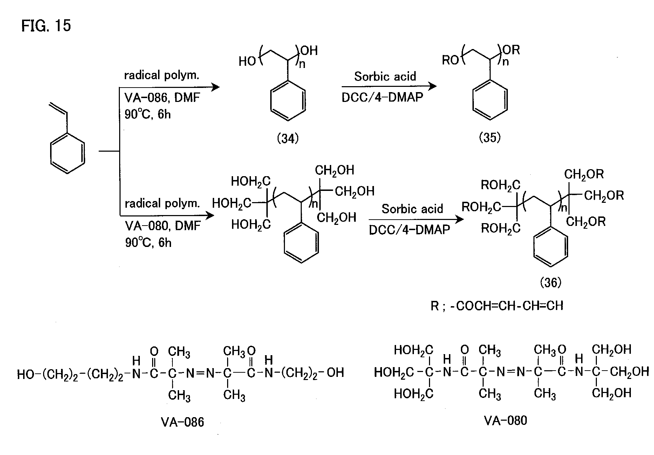

Moreover, a terminal diene polystyrene (35) was synthesized according to the reaction path illustrated in the upper portion of Fig. 15. In a glass tube, 0.2g of VA-086 (commercially available initiating agent), 2.0g of styrene, and 5ml of dimethylformamide (DMF) were introduced, and subjected to 3 cycles of repeating freezing and melting in vacuum, thereby to remove oxygen in the system. Then, the glass tube was sealed. Then, the mixture was reacted at 90°C for 6 hours, and precipitated in a large amount of methanol. The polymer thus obtained was re-precipitated thereby to purify.

A dichloromethane solution (20ml) of dicyclohexylcarbodiimide (DCC) (5.0g) was dropped into a dichloromethane solution (25ml) containing 1g of the polystyrene (34) having a hydroxy group at each end of its chain, sorbic acid (2.0g), and 4-dimethylaminopyridine (4-DMAP) (catalyst quantity (0.2g)). Then, the resultant solution was stirred overnight, precipitating a urea compound, which was then filtered off. The filtrate was concentrated. Then, precipitation was carried out in a large amount of methanol, thereby obtaining diene-terminal polystyrene (35). The diene-terminal polystyrene (35) was then purified by re-precipitation. The resultant diene-terminal polystyrene (telechelic polymer) (35) was powder at an ordinary temperature and its structure was confirmed with ¹H-NMR and ¹³C-NMR.

¹H -NMR (400 MHz, CDCl₃) δ 7.30-6.85 and 6.65-6.40 (broad, Ar, 5H x n), 6.21-6.09 (m, CH₃CH=CH, 2H), 5.73 (d, J = 16.0 Hz, 1H), 2.15-1.70 (broad, CH₂CHPh, 1H x n), 1.86 (d, J = 6.4 Hz, CH₃CH=CH, 3H), 1.60-1.20 (broad, CH₂CHPh, 2H x n); ¹³C-NMR (100 MHz, CDCl₃) δ 167.1 (C=O), 146.0-145.1 (Ar and CH=CHCO), 140.1 (CH₃CH=CH), 129.6 (CH₃CH=CH), 128.0-127.3 and 125.6-125.5 (Ar), 118.2 (CH=CHCO), 44.1-41.8 (CH₂CHPh), 39.1 (CH₂CHPh), 18.7 (CH₃CH=CH)

In a similar manner, a telechelic polymer (36) having 6 diene groups at maximum was synthesized along the reaction path illustrated in the lower portion of Fig. 15, where instead of VA-086, VA-080 was used. The telechelic polymer (36) thus obtained was powder at an ordinary temperature and whose structure was confirmed with ¹H-NMR and ¹³C-NMR.

¹H-NMR (400 MHz, CDCl₃) δ 7.28-6.83 and 6.70-6.30 (broad, Ar, 5H x n), 6.20-6.09 (m, CH₃CH=CH, 2H), 5.70 (d, J = 14.8 Hz, 1H), 2.25-1.70 (broad, CH₂CHPh, 1H x n), 1.85 (d, J = 3.2 Hz, CH₃CH=CH, 3H), 1.60-1.25 (broad, CH₂CHPh, 2H x n); ¹³C-NMR (100 MHz, CDCl₃) δ166.9 (C=O), 146.0-145.1 (Ar and CH=CHCO), 140.3 (CH₃CH=CH), 129.6 (CH₃CH=CH), 128.0-127.3 and 125.6-125.4 (Ar), 117.9 (CH=CHCO), 45.9-41.6 (CH₂CHPh), 40.3 (CH₂CHPh), 18.7 (CH₃CH=CH)

Copolymerization of the diene-terminal polystyrene (1.0g) and oxygen was carried out in 1,2-dichloroethane at 30°C for 12 hours with AMVN (0.02g) as an initiating agent, blowing oxygen therein. Moreover, in order to promote production of polyperoxide, the AMVN (0.02g) was added every 2 hours. Thereby, a degradable polymer having a gel structure was obtained, which swelled in solvents such as toluene, THF, and the like, which are good solvent for polystyrene.

Next, thermolysis of the degradable polymer having the gel structure was carried out. The degradable polymer (obtained from a diene-terminal polystyrene of a number average molecular weight of 3.1 x 10⁴ and oxygen) having the gel structure swollen in toluene was heated at 100°C. The heat treatment for 10 minutes caused flowability of the degradable polymer and solubility thereof in a solvent due to cleavage of the peroxide bonding. After the decomposition, the toluene solution was purred into a large amount of methanol, thereby precipitating polystyrene. GPC measurement of the polystyrene showed that it has a number average molecular weight of 4.9 x 1_{04.}

Moreover, it was confirmed that addition of triethylamine decomposed the gel. The product of the decomposition had a number average molecular weight of 4.7 x 10⁴. The present Example confirmed that a gel-structured degradable polymer having a peroxide bond at cross-linking point was synthesized from the diene-terminal polystyrene and oxygen, and that heat treatment or addition of a base cleaved the peroxide bonding thereby solating the degradable polymer.

The diene group for copolymerization with oxygen does not need to be at the terminal of the polymer. Two or more diene groups present in the side chain are sufficient to synthesize such a gel. Such a polymer having diene group in its side chain can be synthesized from reaction of a polymer or copolymer having a hydroxyl group in its side chain (such as polyvinylalcohol, 2-hydroxyethyl polymethacrylate, and the like) with sorbic chloride or sorbic isocyanate. The functional group is not limited to hydroxyl group. Any group capable of being easily converted to diene group by polymerization can be used for this purpose similarly.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

### INDUSTRIAL APPLICABILITY

The present invention is applicable in the medical field, and medical material filed, adaptable to DDS and gene delivery system, and usable in a novel polymer material and environmentally-friendly material.

## Claims

1. A degradable polymer having in its main chain a peroxide bond by radical alternating copolymerization of a diene monomer and oxygen, the degradable polymer comprising:
a substituent in its side chain, the substituent having at least one function selected from the group consisting of a pharmacologic activity function, an optical function, an electronic function, an electric function, a magnetic function, a chiral recognizing function, a catalyst function, a liquid crystal function, an actuator function, and a sensor function.

2. The degradable polymer as set forth in claim 1, having a structure represented by General Formula (I): 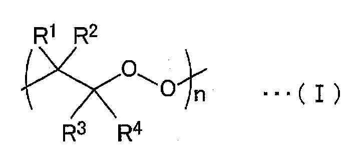 where R¹, R², and R³ are independently an alkyl group or an aromatic group, and R⁴ is a substituent having at least one function selected from the group consisting of the pharmacologic activity function, the optical function, the electronic function, the electric function, the magnetic function, the chiral recognizing function, the catalyst function, the liquid crystal function, the actuator function, and the sensor function, and n is any integer.

3. The degradable polymer as set forth in claim 1, having a structure represented by General Formula (II): 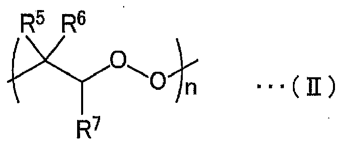 where R⁵ and R⁶ are independently an alkyl group or an aromatic group, and R⁷ is a substituent having at least one function selected from the group consisting of the pharmacologic activity function, the optical function, the electronic function, the electric function, the magnetic function, the chiral recognizing function, the catalyst function, the liquid crystal function, the actuator function, and the sensor function, and n is any integer.

4. The degradable polymer as set forth in claim 1, having a structure represented by General Formula (III): 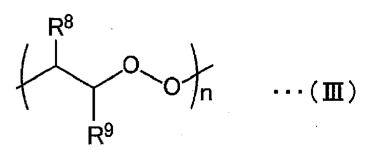 where R⁸ is an alkyl group or an aromatic group, and R⁹ is a substituent having at least one function selected from the group consisting of the pharmacologic activity function, the optical function, the electronic function, the electric function, the magnetic function, the chiral recognizing function, the catalyst function, the liquid crystal function, the actuator function, and the sensor function, and n is any integer.

5. The degradable polymer as set forth in claim 1, wherein the substituent is a drug molecule.

6. The degradable polymer as set forth in claim 1, wherein the substituent is water soluble.

7. The degradable polymer as set forth in claim 1, wherein the substituent is biodegradable.

8. The degradable polymer as set forth in claim 1, wherein the diene monomer includes a compound having at least two diene groups.

9. The degradable polymer as set forth in claim 8, having a gel structure.

10. A method of producing a degradable polymer having in its main chain a peroxide bond by radical alternating copolymerization of a diene monomer and oxygen, the degradable polymer including a substituent in its side chain, the substituent having at least one function selected from the group consisting of a pharmacologic activity function, an optical function, an electronic function, an electric function, a magnetic function, a chiral recognizing function, a catalyst function, a liquid crystal function, an actuator function, and a sensor function, the method comprising:
performing the radical alternating copolymerization after bonding the diene monomer with the substituent, so as to have the structure in which the degradable polymer includes the substituent in its side chain.

11. The method as set forth in claim 10, wherein the substituent is at least one selected from the group consisting of a substituent made of a drug molecule, a water soluble substituent, and a biodegradable substituent.

12. A method of producing a degradable polymer having in its main chain a peroxide bond by radical alternating copolymerization of a diene monomer and oxygen, the degradable polymer including a substituent in its side chain, the substituent having at least one function selected from the group consisting of a pharmacologic activity function, an optical function, an electronic function, an electric function, a magnetic function, a chiral recognizing function, a catalyst function, a liquid crystal function, an actuator function, and a sensor function, the method comprising:
bonding the substituent with the degradable polymer in its side chain after performing the radical alternating copolymerization of the diene monomer with the substituent.

13. The method as set forth in claim 12, wherein the substituent is at least one selected from the group consisting of a substituent made of a drug molecule, a water soluble substituent, and a biodegradable substituent.

14. A method of producing a degradable polymer having in its main chain a peroxide bond by radical alternating copolymerization of a diene monomer and oxygen, the degradable polymer including a substituent in its side chain, the substituent having at least one function selected from the group consisting of a pharmacologic activity function, an optical function, an electronic function, an electric function, a magnetic function, a chiral recognizing function, a catalyst function, a liquid crystal function, an actuator function, and a sensor function, wherein:
the diene monomer includes a compound having at least two diene groups.
